(19) Europäisches Patentamt
European Patent Office
Office européen des brevets

(11) **EP 4 157 125 B1**

(12) **EUROPEAN PATENT SPECIFICATION**

(45) Date of publication and mention
of the grant of the patent:
**02.04.2025 Bulletin 2025/14**

(51) International Patent Classification (IPC):
**A61B 18/20** (2006.01)   **A61N 5/06** (2006.01)
**A61B 18/00** (2006.01)

(21) Application number: **21725781.5**

(22) Date of filing: **19.05.2021**

(52) Cooperative Patent Classification (CPC):
**A61B 18/203; A61N 5/0617;** A61B 2018/00458;
A61B 2018/0047; A61B 2018/00476;
A61B 2018/00904

(86) International application number:
**PCT/EP2021/063233**

(87) International publication number:
**WO 2021/239529 (02.12.2021 Gazette 2021/48)**

(54) **APPARATUS FOR PROVIDING VISUAL GUIDANCE FOR A USER OF A PERSONAL CARE DEVICE**

VORRICHTUNG ZUR BEREITSTELLUNG EINER VISUELLEN FÜHRUNG FÜR EINEN BENUTZER EINER KÖRPERPFLEGEVORRICHTUNG

APPAREIL POUR FOURNIR UN GUIDAGE VISUEL À UN UTILISATEUR D'UN DISPOSITIF DE SOINS PERSONNELS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2020 EP 20177086**

(43) Date of publication of application:
**05.04.2023 Bulletin 2023/14**

(73) Proprietor: **Koninklijke Philips N.V.**
**5656 AG Eindhoven (NL)**

(72) Inventors:
• **KOOIJMAN, Gerben**
**5656 AE Eindhoven (NL)**
• **FERNANDO, Shakith Devinda**
**5656 AE Eindhoven (NL)**
• **BROUWER, Jan**
**5656 AE Eindhoven (NL)**
• **VAN BREE, Karl Catharina**
**5656 AE Eindhoven (NL)**
• **DE BRUIJN, Frederik Jan**
**5656 AE Eindhoven (NL)**
• **VAN DEN ELZEN, Stefano Johannes**
**5656 AE Eindhoven (NL)**
• **HEINRICH, Adrienne**
**5656 AE Eindhoven (NL)**

(74) Representative: **Philips Intellectual Property & Standards**
**High Tech Campus 52**
**5656 AG Eindhoven (NL)**

(56) References cited:
WO-A1-2017/083017    WO-A1-2019/224273
WO-A1-2019/224276    US-A1- 2018 204 346

**Description**

FIELD OF THE INVENTION

This disclosure relates to a personal care device for performing a personal care operation of a body of a subject, and in particular relates to a method and apparatus for providing visual guidance to a user of the personal care device on movement of the personal care device across a body of a subject.

BACKGROUND OF THE INVENTION

Techniques for removal of unwanted hairs include shaving, electrolysis, plucking, laser and light therapies (known as photoepilation) and injection of therapeutic anti-androgens. Light-based technologies are also used in other types of dermatological treatments, including hair growth reduction and treating acne.

Through the use of an appropriate configuration of the light energy, i.e. in terms of wavelength, intensity and/or pulse duration (if the light is to be pulsed), selective heating of the hair root and subsequent temporary or permanent damage to the hair follicle can be achieved. Home-use photoepilation devices, for example the Philips Lumea device, use intense pulsed light (IPL) from high intensity light sources, e.g. Xenon flash lamps that produce high output bursts of broad spectrum light.

A photoepilation treatment is characterized by the user of the photoepilation device treating relatively small areas of the skin for the purpose of hair removal. The photoepilation treatment uses intense light to heat melanin in hair and hair roots, which puts the hair follicles into a resting phase, preventing hair re-growth. For effective use of this technology for hair removal, the user must treat the skin completely without leaving any gaps. Since this effect is only of limited duration, treatment has to be repeated on a regular basis: typically once every 4 to 8 weeks in the maintenance phase after an initial period of about two months in which treatment is performed once every two weeks.

In a typical photoepilation treatment, the user of the photoepilation device must repeatedly manually position the photoepilation device on the skin and trigger a light pulse in order to cover a full body part (e.g. an arm, a leg, etc.). In order to obtain optimal results from the photoepilation treatment, the user must obtain high skin coverage during use, i.e. most of the skin surface must be treated with the device. However, as a photoepilation device typically does not provide any feedback to the user about the areas that have already been treated, and there are little or no user-perceptible changes to the skin or hairs on applying a light pulse or shortly after applying a light pulse, it is difficult for a user to achieve complete coverage of a body part and/or avoid over-treating certain areas of the body part.

In addition, users find it difficult to cover the entire skin surface during a photoepilation treatment as it is difficult to estimate how much the photoepilation device needs to be moved between flashes. This is related to the fact that in some photoepilation devices the actual treatment window (the optical output window) of the photoepilation device is relatively small and it is surrounded by a substantial edge: i.e. the flash window is considerably smaller than the photoepilation device's nozzle which is in contact with the skin. Furthermore, there is no guidance feedback to the user of the photoepilation device of previously treated areas. The absence of any indication of the treated areas poses a challenge to the consumer to achieve effective treatment coverage. In the absence of localised visual or tactile feedback, it is difficult to achieve optimal coverage of the treated area during successive exposures (flashes). This can both lead to under- or over-treatment of the area, both of which are undesirable for different reasons. In the case of under-treatment, body areas are only partly covered, giving unwanted hair regrowth on areas that have not been treated. This can lead to consumer dissatisfaction, reduced trust in the correct usage of the photoepilation device, and a risk of perceived low quality/poor functioning of photoepilation products. In the case of over-treatment, this can lead to irritation to the skin through excessive exposure to the light.

Similar problems apply to other types of personal care devices for performing other types of the personal care operation where there is no or little visible (or otherwise user-perceptible) indication of areas that have already been treated, or that still need to be treated.

WO 2019/224273 A1 discloses a device for treating a human body part with light pulses. The device comprises a handpiece for applying light pulses to the body part. The handpiece is movable relative to the body part and comprises an exit window via which the light pulses are applied to a localised area of the body part. The handpiece further comprises a motion detector. The device also comprises a system for controlling the emission of the light pulses which is configured so as to automatically trigger the light pulses during the movement of the handpiece over the boy part at a rate determined by at least one piece of information provided by the motion detector. The device further comprises a projection system for projecting guidance information on the body part in order to guide the operator of the device during the treatment and/or information to help locating the handpiece. In particular, the projection system at least partially illuminates the body part in order to differentiate the treated areas from the untreated areas of the body part. Alternatively or in addition, the projection system produces an image on the body part configured to differentiate the areas of the skin by the number of passages of the handpiece over each area of skin.

Therefore it is desirable to be able to provide visual guidance to a user of a personal care device on a personal care operation to be performed on a body of a subject to improve the coverage of the body and/or improve the effectiveness of the personal care operation on the body.

SUMMARY OF THE INVENTION

Embodiments of the techniques described herein aim to provide visual guidance to a user of a personal care device on movement of the personal care device across a body of a subject.

According to a first specific aspect, there is provided an apparatus configured for use with a personal care device. The apparatus is configured to provide visual guidance to a user on movement of the personal care device across a body of a subject, wherein the personal care device is configured to perform a personal care operation on a plurality of successive treatment areas of the body by successively moving the personal care device from a respective one of a plurality of successive current treatment positions on the body corresponding with a respective one of said successive treatment areas to a next treatment position on the body corresponding with a next one of said successive treatment areas and performing the personal care operation to said next one of said successive treatment areas while maintaining the personal care device in said next one of said successive treatment positions. The apparatus comprises a projection unit configured to project a light pattern on to a surface of the body and a processing unit. The processing unit is configured to control the projection unit to project the light pattern on to the surface of the body in a projection position indicative of the next treatment position on the body to which the personal care device is to be moved by the user from the current treatment position on the body.

In some embodiments, the projection position is such that, seen in a direction in which the personal care device is to be moved by the user, the treatment area corresponding with the next treatment position seamlessly connects to the treatment area corresponding with the current treatment position. In this way, the likelihood of gaps being left between successive treatment areas is reduced.

In some embodiments, the processing unit is configured to control the projection unit to project the light pattern in the projection position after the personal care operation has been performed at the current treatment position of the personal care device on the body. In this way, the visual guidance is provided at the time the personal care device is to be moved to the next treatment position.

In some embodiments, the processing unit is configured to control the projection unit to project the light pattern in response to a first input from the user. These embodiments provide the user with control over when the light pattern is to be projected.

In some embodiments, the processing unit is configured to control the projection unit to stop the projection of the light pattern in response to a second input from the user. These embodiments enable the user to deactivate the projection of the light pattern, for example when the user is about to move the personal care device once they have visually memorised where the personal care device is to be moved to next.

In some embodiments, the processing unit is further configured to receive a signal from a movement sensor associated with the personal care device; process the received signal to determine when the personal care device starts to move from the current treatment position on the body; and adjust the operation of the projection unit when the personal care device is determined to be starting to move from the current treatment position. **In** these embodiments, the processing unit can be configured to adjust the operation of the projection unit by controlling the projection unit to deactivate the projection of the light pattern when the personal care device starts to move from the current treatment position. These embodiments provide for the automatic deactivation of the projection of the light pattern so that the user does not have to remember do this, and also provides that the light pattern is not moved across the surface of the body with the movement of the personal care device, which might confuse the user as to the correct next treatment position. Alternatively, the processing unit can be configured to control the projection unit based on the signal received from the movement sensor such that the projection position of the projected light pattern is substantially static relative to the surface of the body in response to the movement of the personal care device from the current treatment position to the next treatment position. These embodiments provide for the light pattern to be projected at the correct position on the body even as the personal care device is moved towards that position.

In some embodiments, the light pattern comprises one or more lines indicative of a position of an edge of the personal care device on the surface of the body associated with the next treatment position of the personal care device on the body.

In alternative embodiments, the light pattern is configured to highlight the treatment area of the body corresponding with the next treatment position of the personal care device on the body.

In some embodiments, the processing unit is further configured to (i) receive a signal from a movement sensor associated with the personal care device; (ii) process the received signal to determine an amount of movement of the personal care device from a first treatment position at which a first personal care operation is performed by the personal care device to a next, second, treatment position at which a second personal care operation is performed by the personal care device; (iii) repeat (ii) for a plurality of movements of the personal care device from first treatment positions to next, second, treatment positions; and (iv) compare each determined amount of movement to an expected amount of movement from each first treatment position to the respective next second treatment position; wherein the processing unit is configured to control the projection unit to project the light pattern on to the surface of the body in a projection position indicative of a next treatment position on the body to which the personal care device is to be moved based on the result of the comparison. These embodiments provide the advantage that the projection of the light pattern can be adapted in view of previous errors in the distance moved by the user to the next treatment position so that the likelihood of the user moving the personal care device to the correct position is increased.

In some embodiments, (i) the apparatus further comprises at least one further projection unit configured to project a further light pattern on to a different part of the body; or (ii) the projection unit is further configured to project a further light pattern on to a different part of the body.

According to a second aspect, there is provided a personal care device configured to perform a personal care operation on a body of a subject. The personal care device comprises an apparatus according to the first aspect or any embodiment thereof configured to provide visual guidance to a user of the personal care device on movement of the personal care device across the body of the subject.

According to a third aspect, there is provided a method of operating an apparatus to provide visual guidance to a user of a personal care device. The visual guidance is for guiding movement of the personal care device across a body of a subject by the user, wherein the personal care device is configured to perform a personal care operation on a plurality of successive treatment areas of the body by successively moving the personal care device from a respective one of a plurality of successive current treatment positions on the body corresponding with a respective one of said successive treatment areas to a next treatment position on the body corresponding with a next one of said successive treatment areas and performing the personal care operation to said next one of said successive treatment areas while maintaining the personal care device in said next one of said successive treatment positions. The method comprises controlling a projection unit in the apparatus to project a light pattern on to the surface of the body in a projection position indicative of the next treatment position on the body to which the personal care device is to be moved by the user from the current treatment position on the body.

In some embodiments, the projection position is such that, seen in a direction in which the personal care device is to be moved by the user, the treatment area corresponding with the next treatment position seamlessly connects to the treatment area corresponding with the current treatment position. In this way, the likelihood of gaps being left between successive treatment areas is reduced.

In some embodiments, the step of controlling comprises controlling the projection unit to project the light pattern in the projection position after the personal care operation has been performed at the current treatment position of the personal care device on the body. In this way, the visual guidance is provided at the time the personal care device is to be moved to the next treatment position.

In some embodiments, the step of controlling comprises controlling the projection unit to project the light pattern in response to a first input from the user. These embodiments provide the user with control over when the light pattern is to be projected.

In some embodiments, the step of controlling comprises controlling the projection unit to stop the projection of the light pattern in response to a second input from the user. These embodiments enable the user to deactivate the projection of the light pattern, for example when the user is about to move the personal care device once they have visually memorised where the personal care device is to be moved to next.

In some embodiments, the method further comprises receiving a signal from a movement sensor associated with the personal care device; processing the received signal to determine when the personal care device starts to move from the current treatment position on the body; and adjusting the operation of the projection unit when the personal care device is determined to be starting to move from the current treatment position. In these embodiments, the operation of the projection unit can be adjusted so that the projection unit deactivates the projection of the light pattern when the personal care device starts to move from the current treatment position. These embodiments provide for the automatic deactivation of the projection of the light pattern so that the user does not have to remember do this, and also provides that the light pattern is not moved across the surface of the body with the movement of the personal care device, which might confuse the user as to the correct next treatment position. Alternatively, the operation of the projection unit can be controlled based on the signal received from the movement sensor so that the projection position of the projected light pattern is substantially static relative to the surface of the body in response to the movement of the personal care device from the current treatment position to the next treatment position. These embodiments provide for the light pattern to be projected at the correct position on the body even as the personal care device is moved towards that position.

In some embodiments, the light pattern comprises one or more lines indicative of a position of an edge of the personal care device on the surface of the body associated with the next treatment position of the personal care device on the body.

In alternative embodiments, the light pattern highlights the treatment area of the body corresponding with the next treatment position of the personal care device on the body.

In some embodiments, the method further comprises (i) receiving a signal from a movement sensor associated with the personal care device; (ii) processing the received signal to determine an amount of movement of the personal care device from a first treatment position at which a first personal care operation is performed by the personal care device to a next, second, treatment position at which a second personal care operation is performed by the personal care device; (iii) repeating (ii) for a plurality of movements of the personal care device from first treatment positions to next, second, treatment positions; and (iv) comparing each determined amount of movement to an expected amount of movement from each first treatment position to the respective next second treatment position; wherein the processing unit is configured to control the projection unit to project the light pattern on to the surface of the body in a projection position indicative of a next treatment position on the body to which the personal care device is to be moved based on the result of the comparison. These embodiments provide the advantage that the projection of the light pattern can be adapted in view of previous errors in the distance moved by the user to the next treatment position so that the likelihood of the user moving the personal care device to the correct position is increased.

In some embodiments, (i) the method further comprises controlling at least one further projection unit to project a further light pattern on to a different part of the body; or (ii) controlling the projection unit to project a further light pattern on to a different part of the body.

According to a fourth aspect, there is provided a computer program product comprising a computer readable medium having computer readable code embodied therein, the computer readable code being configured such that, on execution by a suitable computer or processor, the computer or processor is caused to perform the method according to the third aspect or any embodiment thereof.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Figs. 1(a) and 1(b) are illustrations of two exemplary personal care devices according to various embodiments;
Fig. 2 is a block diagram of an exemplary system comprising an apparatus according to various embodiments;
Fig. 3 shows an infra-red image of an arm after a number of light pulses have been applied;
Fig. 4 illustrates successive treatment areas on a part of the body;
Figs. 5(a) and 5(b) illustrate a projection of a light pattern on to a surface of a body according to various embodiments;
Fig. 6 illustrates the use of an embodiment of a personal care device according to the techniques described herein;
Fig. 7 illustrates changes in the projected light pattern as the personal care device moves according to various embodiments;
Fig. 8 illustrates a personal care device and a light pattern according to an embodiment;
Fig. 9 illustrates a personal care device and light pattern according to another embodiment; and
Fig. 10 is a flow chart illustrating an exemplary method according to the techniques described herein.

DETAILED DESCRIPTION OF THE EMBODIMENTS

As noted above, the techniques described herein can be used to provide visual guidance to a user of a personal care device on movement of the personal care device across a body of a subject to perform a personal care operation on the body. In particular, a projection unit is used to project a light pattern on to the body to indicate a next treatment position on the body that the personal care device is to be moved to by the user.

An apparatus is described that implements the techniques described herein. The apparatus comprises a processing unit and a projection unit for providing the visual guidance. In some embodiments, the apparatus is separate from the personal care device, and the apparatus and the personal care device form a system. In other embodiments, the apparatus is part of the personal care device. In other embodiments, the processing unit of the apparatus is part of the personal care device and the projection unit of the apparatus is separate from the personal care device. In embodiments where the apparatus (or at least the processing unit) is separate from the personal care device, the apparatus can be in the form of, e.g. a smartphone, a tablet, a smartwatch, a laptop, a computer, a server, etc.

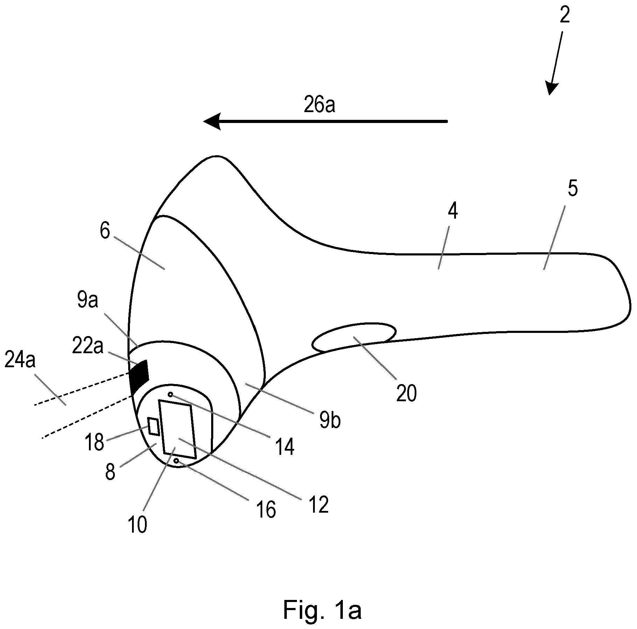

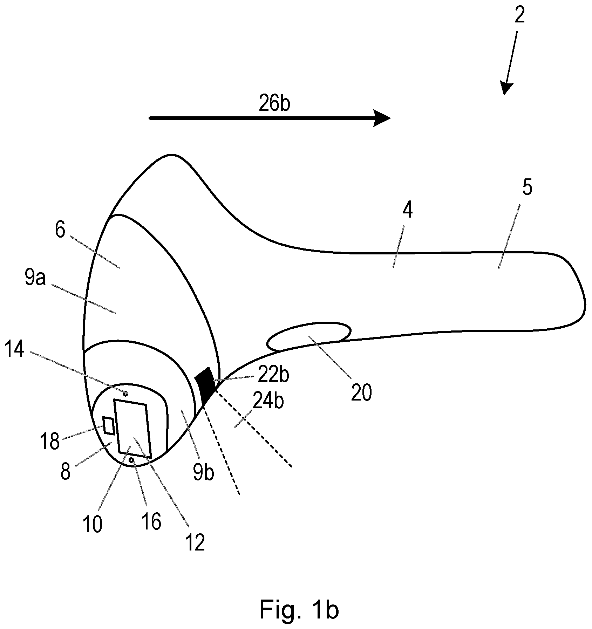

Figs. 1(a) and 1(b) illustrate two embodiments of a personal care device according to the techniques described herein. In the embodiments shown in Fig. 1, the apparatus is part of the personal care device. The embodiments shown in Figs. 1(a) and 1(b) are generally similar, except for the positions of the projection unit in the housing of the personal care device. References below generally to Fig. 1 refer to both Fig. 1(a) and Fig. 1(b).

Thus Fig. 1 is an illustration of an exemplary personal care device 2 that can be used to apply energy or an energy pulse (e.g. light, or a light pulse) to an area of skin. It will be appreciated that the personal care devices 2 in Fig. 1 are merely presented as an example of a hand-held personal care device 2 that the invention can be used with, and the personal care device 2 is not limited to the forms shown in Fig. 1 or to being a hand-held personal care device. The personal care device 2 is for use on a body of a subject (e.g. a person or an animal), and is to be held in one or both hands of a user during use. The personal care device 2 is to perform some personal care operation to the skin or body of the subject using energy (e.g. light) or one or more energy pulses (e.g. light pulses) when the personal care device 2 is in contact with, or close to, a body part of the subject. The personal care operation can be removal of unwanted hairs by laser and/or light therapies (known as a photoepilation treatment or Intense Pulsed Light, IPL, treatment). Alternatively the personal care operation can be laser and/or light therapies for reasons other than removal or preventing growth of hair, such as a dermatological (skin) treatment, treating acne, a phototherapy treatment for other types of skin condition, skin rejuvenation, skin tightening, or port-wine stain treatment. A personal care operation is also referred to as a 'treatment operation' herein.

As described herein, the personal care device 2 is operated or used by a 'user', and the personal care device 2 is used on a body of a 'subject'. In some cases the user and the subject is the same person, i.e. the personal care device 2 is held in a hand and used by a user on themselves (e.g. used on the skin on their leg). In other cases the user and the subject are different people, e.g. the personal care device 2 is held in a hand and used by a user on someone else. In either case, it is difficult for a user to achieve complete coverage of a body part and/or avoid over-treating certain areas of the body part since there are little or no user-perceptible changes to the skin on applying or shortly after applying energy or an energy pulse.

The exemplary personal care device 2 comprises a housing 4 that includes at least a handle portion 5 and a head portion 6. The handle portion 5 is shaped to enable the user to hold the personal care device 2 with one hand. The head portion 6 has a head end 8, and the head end 8 is to be placed into contact with the subject in order for the personal care operation to be performed on the body or skin of the subject at the position that the head end 8 is in contact with the body or skin. The head portion 6 has a top surface 9a and a bottom surface 9b. The top surface 9a is on a part of the head portion 6 facing generally away from the handle portion 5, and the bottom surface 9b is on the opposite part of the head portion 6, facing generally towards the handle portion 5.

The personal care devices 2 shown in Fig. 1 are for performing a personal care operation using light pulses, and rest of the description of Fig. 1 will refer to light pulses. However, it will be appreciated that the techniques described herein are more generally applicable to the use of light, energy or energy pulses for effecting the personal care operation. Thus, in Fig. 1 the head portion 6 comprises an aperture 10 arranged in or on the housing 4 so that the aperture 10 can be placed adjacent to or on (i.e. in contact with) the skin of the subject. The personal care device 2 includes one or more light sources 12 that are for generating light pulses that are to be applied to the skin of the subject via the aperture 10 and effect a personal care operation. The one or more light sources 12 are arranged in the housing 4 so that the light pulses are provided from the one or more light sources 12 through the aperture 10. The aperture 10 may be in the form of an opening at the head end 8 of the housing 4, or it may be in the form of a window (including a waveguide) that is transparent or semi-transparent to the light pulses (i.e. the light pulses can pass through the window). The area of a part of the body that is treated by a light pulse passing through the aperture 10 is referred to herein as a 'treatment area'.

In the exemplary embodiment shown in Fig. 1, the aperture 10 has a generally rectangular shape, which results in a generally rectangular-shaped skin treatment area on the skin. It will be appreciated that the aperture 10 can have any other desired shape. For example the aperture 10 can be square, elliptical, circular, or any other polygonal shape.

Although not shown in Fig. 1, the personal care device 2 may have one or more removable attachments for the head portion 6 of the personal care device 2 that each includes an aperture 10. The attachments may be for use on different body parts, and have apertures 10 with different sizes. For example one attachment can be for use on a large body part such as the legs and therefore have a large aperture 10, whereas another attachment can be for use on the skin/hair above the upper lip and therefore have a small aperture 10.

The one or more light sources 12 can generate light pulses of any suitable or desired wavelength (or range of wavelengths) and/or intensities. For example, the light source 12 can generate visible light, infra-red (IR) light and/or ultraviolet (UV) light. Each light source 12 can comprise any suitable type of light source, such as one or more light emitting diodes (LEDs), a (Xenon) flash lamp, a laser or lasers, etc. The light source(s) 12 can provide light pulses with spectral content in the 560-1200 nanometre (nm) range for a duration of around 2.5 milliseconds (ms), as these wavelengths heat melanin in the hair and hair root by absorption, which puts the hair follicles in a resting phase, preventing hair regrowth. The intensity of the light pulse should be high enough to effect the personal care operation on the skin or body part adjacent the aperture 10.

The illustrated personal care device 2 also optionally includes two skin contact sensors 14, 16 positioned on or in the head portion 6 that are used to determine whether the head portion 6 is in contact with the skin. The skin contact sensors 14, 16 measure a parameter that is indicative of whether the head portion 6 is in contact with skin, and generate respective measurement signals that comprise a time-series of measurements of the parameter. The measurement signals can be processed to determine if the head portion 6 is in contact with skin. Typically a skin contact sensor is used in a personal care device 2, particularly a photoepilator, to make sure that the personal care device 2 is correctly in contact with skin before a light pulse is generated to avoid the light pulse being directed into the eyes of the user or subject.

In some embodiments the parameter can be capacitance, and so the skin contact sensors 14, 16 can measure capacitance via a respective pair of electrical contacts or electrodes on the surface of the head portion 6, with the measured capacitance being indicative of whether there is skin contact. In alternative embodiments, the parameter can be an intensity or level of light, and so the skin contact sensors 14, 16 can be light sensors that measure an intensity or level of light incident on the light sensor, with the measured intensity or level being indicative of whether there is skin contact (e.g. less/no light could indicate skin contact as the skin obscures the light sensors 14, 16, and vice versa). In other alternative embodiments, the parameter can be a measure of contact pressure, and so the skin contact sensors 14, 16 can measure contact pressure via respective pressure sensors or mechanical switches, with the measured contact pressure being indicative of whether there is skin contact.

The illustrated personal care device 2 also includes an optional skin tone sensor 18 positioned on or in the head portion 6 that can be used to determine a skin tone of the skin that the head portion 6 is in contact with. The skin tone sensor 18 can measure a parameter that is indicative of the skin tone of the skin, and generate a measurement signal that comprises a time-series of measurements of the parameter. The measurement signal can be processed to determine the skin tone of the skin that the head portion 6 is in contact with. Typically a skin tone sensor is used in a personal care device 2, particularly a photoepilator, to make sure that the light pulse has an intensity that is appropriate for the type of skin being treated, or even to prevent a light pulse being generated if the skin type is unsuitable for light pulses (e.g. darker skin which has a much higher melanin content).

In some embodiments the optional skin tone sensor 18 can be a light sensor and the parameter measured by the light sensor can be an intensity or level of light at a particular wavelength or multiple wavelengths reflected from the skin. The measured intensity or level of reflected light at a particular wavelength(s) can be indicative of the skin tone. The measured intensity or level of reflected light can be based on the concentration of melanin in the skin, and thus the measured intensity or level can indicate the melanin concentration. The melanin concentration can be derived, for example, from measurements of light reflection at 660nm (red) and 880nm (infrared) wavelengths.

The illustrated personal care device 2 also includes a user control 20 that can be operated by the user to activate the personal care device 2 so that the head portion 6 performs the required personal care operation on the body of the subject (e.g. the generation of one or more light pulses by the one or more light source(s) 12). The user control 20 may be in the form of a switch, a button, a touch pad, etc.

In the illustrated embodiments, the personal care device 2 also comprises a projection unit 22 configured to project a light pattern on to a surface of the body when the personal care device 2 is in use on a subject. Fig. 1(a) includes an element 22a and Fig. 1(b) includes an element 22b that each illustrate a respective position or location of the projection unit in these embodiments. The projection unit is shown as element 22a in Fig. 1(a) and 22b in Fig. 1(b), although it will be appreciated that in some embodiments the projection unit may be inside the housing 4, in which case elements 22a and 22b may instead be a window or opening in the housing 4 of the personal care device 2 through which the projection unit projects the light pattern.

As noted, the projection unit 22a, 22b is configured to project a light pattern on to a surface of the body (e.g. skin) when the personal care device 2 is in use on a subject, i.e. when the head portion 6 and aperture 10 of the personal care device 2 are in contact with, or otherwise close to, the surface (skin) of the subject. In particular, when the personal care device 2 is positioned at a current treatment position on the body of the subject, the light pattern is to be projected to a projection position on the surface of the body that is indicative of a next treatment position on the body to which the personal care device is to be moved. Thus, the projection unit 22a, 22b is configured in or on the housing 4 so that the light pattern is projected away from the personal care device 2, in a direction that the personal care device 2 is to be moved to the next treatment position.

In the embodiment shown in Fig. 1(a), the projection unit 22a is in the top surface 9a of the housing 4 (i.e. a surface of the housing 4 that is furthest from the handle 5), and projects the light pattern 24a generally in the direction shown by the dashed lines. Thus, as the light pattern 24a indicates the next treatment position for the personal care device 2, the personal care device 2 in Fig. 1(a) is to be moved to the left, as shown by arrow 26a.

In the embodiment shown in Fig. 1(b), the projection unit 22b is in the bottom surface 9b of the housing 4 (i.e. a surface of the housing 4 that is facing generally towards the handle 5), and projects the light pattern 24b generally in the direction shown by the dashed lines. Thus, as the light pattern indicates the next treatment position for the personal care device 2, the personal care device 2 in Fig. 1(b) is to be moved to the right, as shown by arrow 26b.

In some embodiments, the personal care device 2 can comprise projection units 22a, 22b in both surfaces 9a, 9b of the head portion 6 (or the personal care device 2 can comprise a single projection unit 22 that is able to selectively project in one or both of the directions and at a time) so that guidance can be provided when the personal care device 2 is to be moved in either direction (i.e. in direction 26a or 26b).

In some embodiments, the personal care device 2 can comprise two or more projection units 22 arranged around the head end 8 to face different directions in which the personal care device 2 can be moved during a personal care operation, and thereby project the light pattern in a desired direction of movement. Alternatively the personal care device 2 can comprise a single projection unit 22 that can be configured to selectively project out from the personal care device 2 in multiple directions from the head end 8, and thereby project the light pattern in a desired direction of movement.

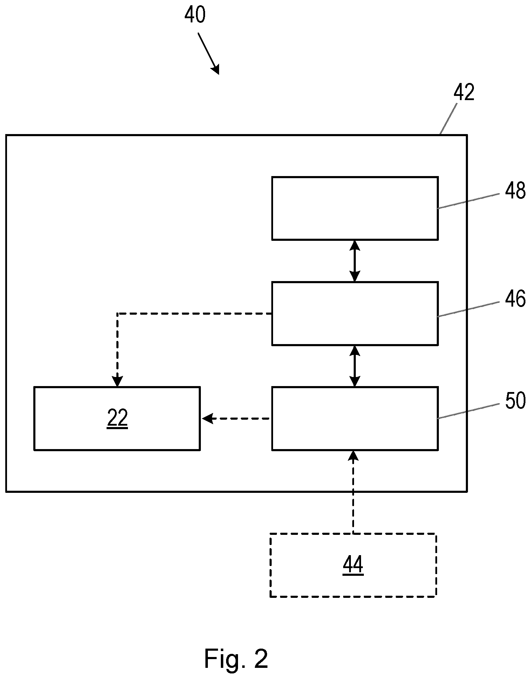

Fig. 2 is a block diagram of an exemplary system 40 comprising an apparatus 42 for providing visual guidance to a user on movement of the personal care device across a body of a subject. In some embodiments, the system 40 may comprise one or more movement sensors 44 for providing a measurement signal comprising information about movements of the personal care device 2 over time. Depending on the implementation of the apparatus 42 and the personal care device 2, the one or more movement sensors 44 may be part of the apparatus 42, or separate from the apparatus 42. In the system 40 shown in Fig. 2, the one or more movement sensors 44 are separate from the apparatus 42.

In some implementations the personal care device 2 can be considered part of the system 40, although the personal care device 2 is not shown in Fig. 2. As noted above, in some embodiments the apparatus 42 can be a separate device to the personal care device 2, and thus the apparatus 42 may be in the form of an electronic device, such as a smart phone, smart watch, tablet, personal digital assistant (PDA), laptop, desktop computer, remote server, smart mirror, etc. In other embodiments, the apparatus 42, and particularly the functionality according to the invention provided by the apparatus 42, is part of the personal care device 2.

In embodiments where the one or more movement sensors 44 are present, a signal (movement signal) from the movement sensor(s) 44 can be processed or analysed to determine any of: if and/or when the personal care device 2 is moving/has moved, a distance that the personal care device 2 is moving/has moved and a direction that the personal care device is moving/has moved.

The one or more movement sensors 44 can include or be an accelerometer, a gyroscope, an air pressure sensor (that can be used for measuring altitude changes), a magnetometer, an ultrasonic sensor, an optical movement sensor, etc., that measure the movements of the personal care device 2 and output a respective measurement signal representing those movements. The one or more movement sensors 44 may be part of or in the personal care device 2. More generally, the one or more movement sensors 44 can be part of an inertial measurement unit (IMU). In the case of the one or more movement sensors 44 comprising an accelerometer, the accelerometer 44 can generate a measurement signal that contains a plurality of acceleration measurement samples representing the movements of the personal care device 2 at a plurality of time instants. The accelerometer 44 may be an accelerometer that measures accelerations in three dimensions, and the measurement signal generated by the accelerometer 44 can include respective measurement signals representing the accelerations in each of the three dimensions. For example, the accelerometer 44 can output respective measurement signals for each of an x-axis, y-axis and z-axis of a Cartesian coordinate system.

The one or more movement sensors 44 can also include or alternatively be an imaging unit (e.g. a camera or an event camera) that obtains a plurality of images or a video sequence. An event camera is an imaging sensor in which each pixel of the sensor independently indicates changes in brightness as they occur. The plurality of images (including images formed from brightness indications from an event camera) or video sequence are output as a measurement signal, and the measurement signal (images/video sequence) can be processed using imaging processing techniques to identify movements of the personal care device 2. In some embodiments, the imaging unit 44 can be attached to or be part of the personal care device 2, in which case the images/video sequence can be processed to extract the movements of the personal care device 2 based on movements of objects (e.g. body parts or skin) visible in the images/video sequence. In other embodiments the imaging unit 44 can be separate from the personal care device 2, e.g. an imaging unit 4 in the apparatus 44, or in a separate device such as a smart phone, smart watch, tablet, personal digital assistant (PDA), laptop, desktop computer, smart mirror, etc. In this case, the imaging unit 44 may be able to observe both the personal care device 2 and the body part from a distance. An imaging unit 44 may include any suitable components for capturing an image, for example a charge-coupled device (CCD) and one or more lenses and/or mirrors. In some embodiments, the imaging unit 44 is a camera, such as a digital camera, or an event camera.

The apparatus 42 comprises the projection unit 22 that configured to project a light pattern on to a surface of the body. The projection unit 22 includes at least one light source (e.g. a light emitting diode (LED), a laser diode, etc.) for generating light. The projection unit 22 may be configured to enable the position and/or other characteristic (e.g. shape, colour, etc.) of the light pattern to be changed. For example the projection unit 22 can include a filter, grating, aperture, lens or other suitable arrangement in front of the light source(s) so that the desired light pattern is created and projected on to the surface of the body. The desired light pattern may be a line, multiple lines, multiple lines arranged in parallel, multiple lines arranged into a shape outline, for example similar to the shape of the aperture 10, a shape, for example a rectangle similar to the shape of the aperture 10, etc. The desired light pattern may have one or more colours, and the projection unit 22 may be configured so that the colour of the light pattern can be changed. In some embodiments, the colour of the light pattern can be changed by using light source(s) that have different colours, whereas in other embodiments the colour of the light pattern can be changed using different coloured light filters.

In some embodiments, the direction/angle that the projection unit 22 projects the light pattern may be fixed. However in other embodiments the projection unit 22 may be controllable to change the direction/angle in which the light pattern is projected. This can enable the position of the light pattern on the surface relative to the personal care device 2 to be adjusted as described further below. In these embodiments, the projection unit 22 may be configured so that the

EP 4 157 125 B1

direction/angle of the light pattern can be adjusted or changed by adjusting a position of a light source in the projection unit 22, by adjusting a position or optical characteristics of any of a filter, grating, aperture, lens or other suitable arrangement in front of the light source(s), or by adjusting the direction/angle of the projection unit 22 in the personal care device itself. In any of these embodiments a suitable actuating mechanism can be provided for adjusting the position of the light source(s), filter, grating, aperture, lens, other suitable arrangement, or the projection unit 22. In some embodiments, the actuating mechanism can include a wheel mechanism or a magnetic glider that can mechanically tilt the projection unit 22 in the personal care device 2.

In some embodiments, the projection unit 22 is a projector, such as a pico-projector or micro-electromechanical system (MEMS) pico-projector.

The apparatus 42 comprises a processing unit 46 that generally controls the operation of the apparatus 42 and enables the apparatus 42 to perform the method and techniques described herein. The processing unit 46 controls the operation of the projection unit 22, and in particular outputs a control signal to the projection unit 22 to control the projection unit 22 to project the light pattern on the surface of the body. In embodiments where the light pattern can be varied, for example during movement of the personal care device 2, the processing unit 46 can control the adjustment of the projected light pattern by the projection unit 22. The processing unit 46 can include or comprise one or more output ports or wires for outputting the control signal for the projection unit 22. The processing unit 46 can include or comprise one or more input ports or wires for receiving measurements or signals from the one or more movement sensors 44. The processing unit 46 can also include or comprise one or more input ports or wires for receiving a trigger signal from the user control 20, or from other control circuitry in the personal care device 2 that indicates when a personal care operation (e.g. light pulse) has been triggered by the user.

The processing unit 46 can be implemented in numerous ways, with software and/or hardware, to perform the various functions described herein. The processing unit 46 may comprise one or more microprocessors or digital signal processors (DSPs) that may be programmed using software or computer program code to perform the required functions and/or to control components of the processing unit 46 to effect the required functions. The processing unit 46 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor) and/or hardware specifically designed for simultaneous localisation and mapping (SLAM) techniques.

The processing unit 46 can comprise or be associated with a memory unit 48. The memory unit 48 can store data, information and/or signals for use by the processing unit 46 in controlling the operation of the apparatus 42 and/or in executing or performing the methods described herein. In some implementations the memory unit 48 stores computer-readable code that can be executed by the processing unit 46 so that the processing unit 46 performs one or more functions, including the methods described herein. In particular embodiments, the program code can be in the form of an application for a smart phone, tablet, laptop, computer or server. The memory unit 48 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 42 may also include interface circuitry 50 to enable a data connection to and/or data exchange with other devices, including any one or more of: the movement sensor(s) 44 (e.g. where the one or more movement sensors 44 are separate from the apparatus 42 - as shown in Fig. 2), the projection unit 22 (e.g. where the projection unit 22 is part of the personal care device 2 but the processing unit 46 is separate from the personal care device 2), more generally the personal care device 2, a consumer electronic device (e.g. a smartphone, tablet, smart watch, laptop, etc.) or remote servers and databases. The interface circuitry 50 may be configured to establish data connections with other components or devices directly or indirectly (e.g. via the Internet), and thus the interface circuitry 50 can enable a connection between the apparatus 42 and a network, or directly between the apparatus 42 and another device (such as movement sensor(s) 44, the projection unit 22 or the personal care device 2), via any desirable wired or wireless communication protocol. For example, the interface circuitry 50 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 50 (and thus apparatus 42) may include one or more suitable antennas for transmitting/receiving over a transmission medium (e.g. the air). Alternatively, in the case of a wireless connection, the interface circuitry 50 may include means (e.g. a connector or plug) to enable the interface circuitry 50 to be connected to one or more suitable antennas external to the apparatus 42 for transmitting/receiving over a transmission medium (e.g. the air). The interface circuitry 50 is connected to the processing unit 46.

Thus, the interface circuitry 50 can enable the apparatus 42/processing unit 46 to receive the measurement signal(s) from the movement sensor(s) 44 (when these sensors 44 are present in the system, 40), and send the projection unit control signal to the projection unit 22 when the projection unit 22 is separate from the processing unit 46.

Although not shown in Fig. 2, the apparatus 42 and/or the personal care device 2 may comprise one or more user interface components that includes one or more components that enables a user of apparatus 42 and/or the personal care device 2 to input information, data and/or commands into the apparatus 42 and/or the personal care device 2, and/or enables the apparatus 42 and/or the personal care device 2 to output information or data to the user of the apparatus 42 and/or the personal care device 2. The user interface can comprise any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display unit or display screen, one or more lights or light elements, one or more loudspeakers, a vibrating element, etc.

It will be appreciated that a practical implementation of an apparatus 42 may include additional components to those shown in Fig. 2. For example the apparatus 42 may also include a power supply, such as a battery, or components for enabling the apparatus 42 to be connected to a mains power supply.

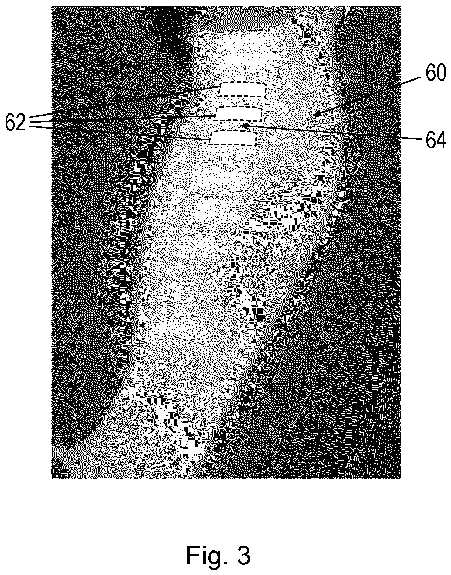

As discussed in the Background section, in a typical light-based treatment the user of the personal care device 2 must manually move the personal care device 2 across the skin to the next treatment position and trigger a light pulse. However, there are little or no user-perceptible changes to the skin or hairs on applying a light pulse or shortly after applying a light pulse, and it is difficult to estimate how much the personal care device 2 needs to be moved between flashes due to differences in size of the actual aperture 10 and the size of the head end 8 of the personal care device 2. This makes it difficult for a user to achieve complete coverage of a body part and/or avoid over-treating certain areas of the body part by repeating a light pulse on an area of skin that has already been treated. To illustrate a typical treatment coverage obtained without any feedback guidance being provided to a user of the personal care device 2, Fig. 3 shows an infra-red image of an arm 60 after a number of light pulses have been applied. This infra-red image was obtained using a thermal camera. It will be appreciated that this image is included for ease of illustrating the problems addressed by the disclosed techniques, and the personal care device 2, apparatus 42 and system 40 do not include a thermal (infra-red) camera. In the image in Fig. 3, the brightness of a part of the image is dependent on the temperature of that part, with brighter (whiter) parts corresponding to higher temperatures and darker (blacker) parts corresponding to lower temperatures. It can be seen that there are a number of bright areas 62 (only some of which are labelled in Fig. 3) on the arm 60, which correspond to areas of higher temperature. These areas have a higher temperature due to a light pulse having recently been applied by a personal care device 2. A light pulse will cause a small (temporary) increase in the temperature of the part of the skin that the light pulse was applied to. It can be seen in Fig. 3 that many parts of the arm 60 have not recently been treated by a light pulse, and specifically there are gaps (labelled 64) between the bright areas 62. However, there is unlikely to be a visible change to the skin caused by the light pulses, so the user of the personal care device 2 will not be able to see which parts of the arm 60 have had light pulses applied. This illustrates the need for guidance for the user of the personal care device 2 so that they are able to identify which part or parts of the body part have already been treated with a light pulse.

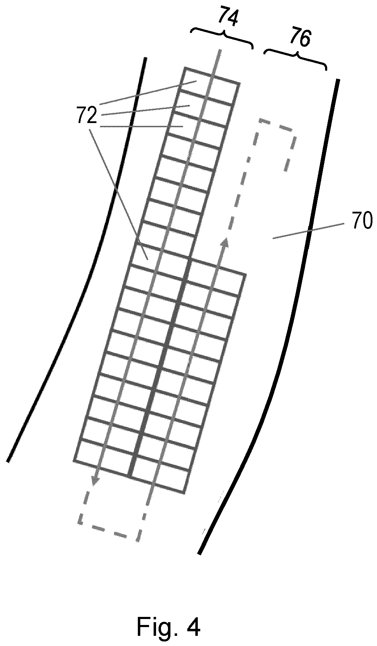

As noted above, a personal care device is placed at a treatment position on the body corresponding to a current treatment area, and a personal care operation (e.g. a light pulse) is applied to the current treatment area while the personal care device is mintained in said treatment position. The personal care device is moved to a new treatment position corresponding to a next treatment area, and the personal care operation performed to the next treatment area while the personal care device is maintained in said new treatment position. This is repeated until the personal care operation has been performed on the desired area of the body (e.g. an arm, a leg, the chest, etc.). For an effective personal care operation, a light pulse should be applied to each part of the desired area of the body. Therefore, typically the user should aim to move the personal care device between the application of light pulses so that the next treatment area is adjacent to (i.e. without a gap or space between the treatment areas), or even partially overlaps with, the previous treatment area. This is illustrated with reference to Fig. 4. Fig. 4 shows a part 70 of a body (e.g. an arm or a leg), and a series of successive treatment areas 72 on the body part 70. In this illustration, the user is to move the personal care device over the body part 70 in a generally straight line and perform the personal care operation so that the successive treatment areas 72 are adjacent to each other. Once the personal care device reaches the end of the desired area, a stroke 74 is completed, and the user repositions the personal care device on the body part 70 to start a new stroke 76. Preferably the treatment areas 72 of the next stroke 76 are adjacent to the treatment areas 72 of the first stroke 74. Although Fig. 4 shows the second stroke 76 starting at the end of the body part 70 that the first stroke 74 ended at, this is merely an example, and the second stroke 76 could start at the same end that the first stroke started at.

Although the treatment areas 72 can be adjacent to each other, it may be desirable for successive treatment areas 72 to partially overlap with each other. This is because the effectiveness of a personal care operation at the edges of the treatment area can be less than in the middle of the treatment area (e.g. due to concentration of the energy of the light pulse in the middle of the aperture 10). In either case, it is useful for the next treatment area to seamlessly connect to a current treatment area.

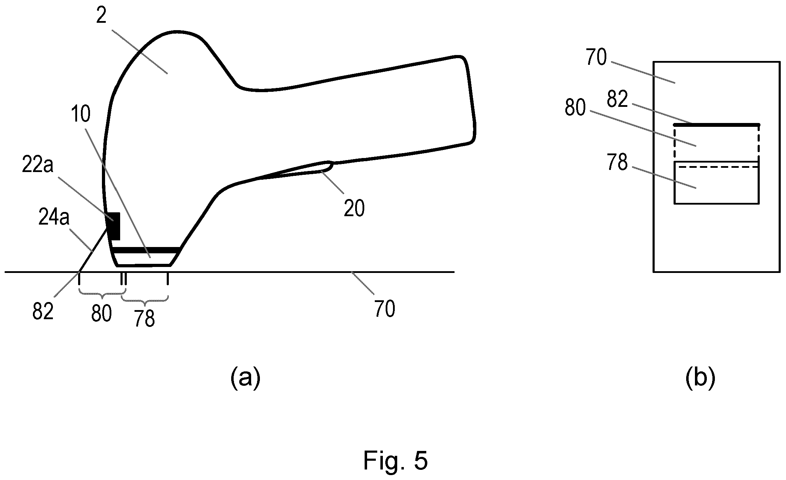

Figs. 5(a) and 5(b) illustrate a projection of a light pattern on to a surface of a body according to various embodiments. Fig. 5(a) shows a side view of a personal care device 2 according to the embodiment in Fig. 1(a) placed on a body part 70 (e.g. an arm, leg, etc.). The personal care device 2 is positioned so that the aperture 10 is adjacent to, or in contact with, the body part 70. At this current position of the personal care device 2, the personal care operation will be performed on a current treatment area 78. After performing the personal care operation on the current treatment area 78, the personal care device 2 is to be moved to the next treatment area 80. In this embodiment, the next treatment area 80 partially overlaps with the current treatment area 78. Fig. 4(b) is a 'top down' view of the surface 70 with the personal care device 2 omitted so that the overlap of the current treatment area 78 and the next treatment area 80 can be more clearly seen.

In accordance with the techniques described herein, the projection unit 22a in the personal care device 2 is used to project a light pattern on to the surface of the body part 70 to provide guidance to the user of the personal care device 2 on the next treatment position of the personal care device 2 on the body part 70. The projection of the light pattern is illustrated by light beam 24a, and the light pattern is projected to a projection position 82 on the body part 70. The projection position 82 is indicative of a next treatment position of the personal care device 2 on the body 70 to which the personal care device 2 is to be moved by the user from the current treatment position on the body 70. In this illustrated embodiment, the projected light pattern is a line that is approximately the same size as an edge of a treatment area, and the projection position 82 corresponds with the edge of the next treatment area 80 that is furthest from the personal care device 2. In this way the user is provided with visual guidance on where the edge of the next treatment area 80 should be, and the user is able to move the personal care device 2 to the correct next treatment position. In an alternative embodiment, particularly where there is a substantial edge around the aperture 10 that means that personal care device 2 is in contact with a larger area of the body part 70 than is treated by the personal care operation, the projection position 82 may indicate where the edge of the head end 8 of the personal care device 2 should be in the next treatment position. In alternative embodiments, rather than projecting a line, the projection unit 22 can project a light pattern to highlight the next treatment area on the body part 70.

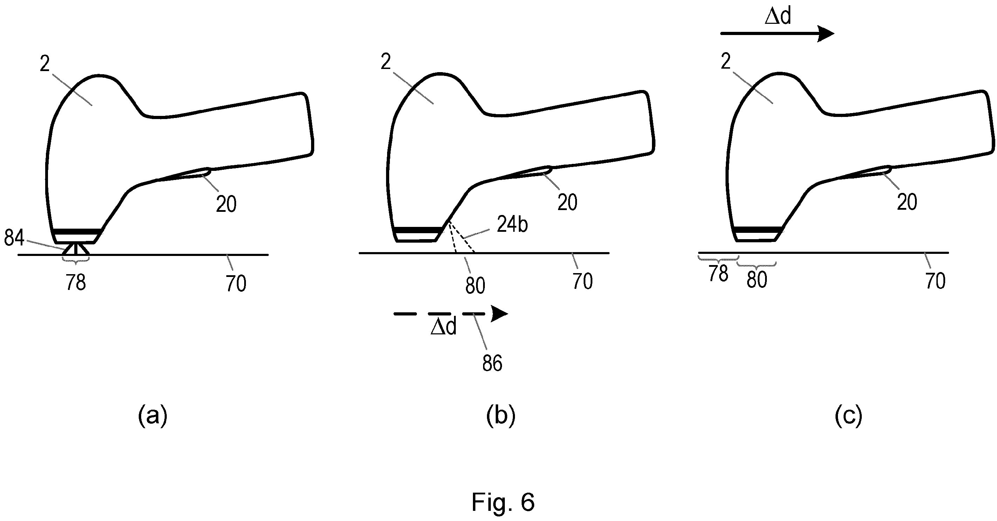

Fig. 6 illustrates the use of an embodiment of a personal care device according to the techniques described herein. Fig. 6 shows a side view of a personal care device 2 according to the embodiment in Fig. 1(b) placed on a body part 70 (e.g. an arm, leg, etc.). In Fig. 6(a), the personal care device 2 is positioned so that the aperture is adjacent to, or in contact with, the body part 70. At this current position of the personal care device 2, the personal care operation will be performed on a current treatment area 78, as shown by the light pulse 84 in Fig. 6(a). The light pulse is triggered in response to a first input by the user. This first input can be the user using the user control 20. After performing the personal care operation on the current treatment area 78, the personal care device 2 is to be moved to the next treatment area 80, which in this embodiment, is to the right in the figure, as shown by dashed arrow 86 in Fig. 6(b). The distance that the personal care device 2 is to be moved is denoted $\Delta d$. For the adjacent treatment areas to be 'seamless', $\Delta d$ should be no longer than a relevant dimension (e.g. height or width) of the aperture 10 in the direction of movement of the personal care device 2. For example, if the aperture 10 has a height of 15 millimetres (mm), then a seamless treatment could be achieved by $\Delta d$ being $\leq 15$mm, e.g. 13mm.

According to the techniques described herein, visual guidance is provided to the user by the projection unit 22b projecting a light pattern 24b as and/or just after the light pulse 84 is applied to the current treatment area 78. This is shown in Fig. 6(b). In some embodiments the light pattern 24b can indicate the edge of the next treatment area 80, similar to the embodiment shown in Fig. 5, and in other embodiments the light pattern 24b can be in the shape of the next treatment area 80, positioned in the appropriate position on the surface of the body part 70. The user can then move the personal care device 2 to the next treatment position indicated by the projected light pattern 24b so that the light pulse can be applied to the next treatment area 80. Therefore the user - on the short time scale needed for moving between treatment positions - memorises visually where the projected light pattern was initially, and moves the personal care device 2 to that next treatment position. This is shown in Fig. 6(c).

In some embodiments, the projection of the light pattern 24b by the projection unit 22a is triggered automatically in response to a first input from the user, e.g. the user actuating user control 20 which triggers the generation of the light pulse 84, or by the generation of the light pulse 84 itself (since, for example, there could be a delay between the user actuating the user control 20 and the generation of the light pulse 84 due to a trigger condition not being met, such as the capacitors in the personal care device 2 not being sufficiently charged, or the skin contact being insufficient, etc.). In alternative embodiments, the user can provide the first input by operating a separate user control that is provided solely for activating the visual guidance by the projection unit 22a, 22b.

In either set of embodiments, the light pattern 24a, 24b may be projected by the projection unit 22a, 22b for a predetermined period of time following the user actuating user control 20, the user actuating a separate dedicated guidance control (e.g. a button or switch), or following the application of the personal care operation (e.g. light pulse) to the current treatment area. For example the light pattern 24a, 24b may be projected for 2 seconds (s), or any other duration sufficient to enable a user of the personal care device 2 to view and understand the amount and/or direction of movement required for the personal care device 2. Alternatively, the light pattern 24a, 24b may be projected by the projection unit 22a, 22b until the user manually provides a second input to deactivate the projection. For example the user may provide the second input to manually deactivate the projection by releasing the user control 20 (e.g. the user control 20 may be a trigger that the user presses or pulls to trigger the light pulse 84, and the light pattern 24a, 24b can be projected until the trigger is released by the user). As another example, the user may provide the second input to manually deactivate the projection by releasing the separate dedicated guidance control, or by actuating a separate guidance deactivation control. In other alternative embodiments, the deactivation of the projection unit 22a, 22b can be based on the movement of the personal care device 2. In particular embodiments the projection unit 22a, 22b can stop projecting the light pattern 24a, 24b when the personal care device 2 starts to move from the current treatment area 78, when the personal care device 2 has moved a minimum distance from the current treatment area 78, and/or when the personal care device 2 is moving at a speed greater than a speed threshold. Thus, in the example of Fig. 6, once the personal care device 2 starts to move towards the next treatment area 80, the light pattern 24b shown in Fig. 6(b) can be deactivated. In the above embodiments, automatically deactivating the light pattern 24a, 24b is beneficial as it can prevent the movement of the projected light pattern 24a, 24b along the body part 70 with the movement of the personal care device 2 from confusing the user as to the correct next treatment position. That is, when the light pattern 24a, 24b is projected from the personal care device 2 in a fixed direction, when the personal care device 2 is moved across the surface of the body part 70 the light pattern 24a, 24b will remain a fixed distance/position ahead of the personal care device 2, which can confuse the user. By automatically deactivating the light pattern 24a, 24b at an appropriate time (e.g. when the personal care device 2 starts to move), the risk of confusion is reduced.

Thus a working principle of embodiments of the projection of the light pattern is that it momentarily points to where the user is to move the personal care device 2 to (i.e. it points to where the user is to move the personal care device 2 until the moment that the user moves the personal care device 2), and that the user - on the short time scale needed for moving between treatment positions - can easily memorise visually where the projected light pattern was.

In embodiments that deactivate the projection of the light pattern when the personal care device 2 moves, the measurements from the one or more movement sensors 44 associated with the personal care device 2 can be analysed by the processing unit 46 to determine when the personal care device 2 is moving. In these embodiments, it is not required to accurately determine the velocity or distance/displacement of the movement, and instead, for example, when the movement sensor(s) 44 comprises an accelerometer, it is sufficient for the processing unit 46 to detect that the measured acceleration (e.g. after acceleration due to gravity is subtracted) is above a certain threshold. More specifically, the processing unit 46 can determine the magnitude of the acceleration, subtracting acceleration due to gravity, and comparing the resulting acceleration to a threshold. If the resulting acceleration is above the threshold, the processing unit 46 can deactivate the projection unit 22a, 22b. Those skilled in the art will be aware of ways in which measurement signals from other types of movement sensors 44 (e.g. an optical movement sensor) can be processed to determine if/when the personal care device is moved. In alternative embodiments, the processing unit 46 can process the measurement signal(s) from the movement sensor(s) 44 to determine a distance or amount of displacement of the personal care device 2, for example by twice integrating the acceleration signal, and/or determine a speed or velocity of movement of the personal care device, for example by integrating the acceleration signal.

In embodiments where the projection unit can project a light pattern in different directions (e.g. in an embodiment of a personal care device 2 that includes both projection units 22a, 22b or a projection unit 22 that is able to project in both directions 24a, 24b), the processing unit 46 can process a measurement signal from a movement sensor 44 to determine the direction that the personal care device 2 is moving, or has previously moved (e.g. determine the direction that the personal care device 2 moved in order to arrive at the current treatment position), and the processing unit 46 can use this information to control the projection of the light pattern accordingly. For example the processing unit 46 can activate the relevant projection unit 22a, 22b to project a light pattern on to the surface in the direction of movement, or the processing unit 46 can control the projection unit 22 to project the light pattern in the direction of movement.

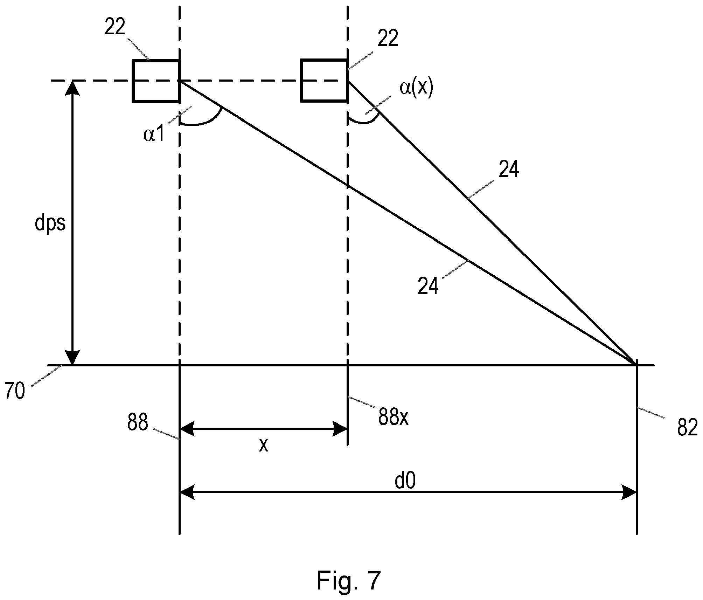

In the above embodiments, the angle of projection of the light pattern 24a, 24b relative to the personal care device 2 is fixed (i.e. once the light pattern 24a, 24b is being projected, it does not change and it is only deactivated). However, in alternative embodiments, the angle of projection of the light pattern 24a, 24b relative to the personal care device 2 can be changed dynamically based on the movement of the personal care device 2 towards the next treatment area 80 so that the visual guidance provided to the user is substantially static relative to the surface of the body part 70 throughout the movement of the personal care device 2 to the next treatment position. That is, in these embodiments, before the personal care device 2 is moved from the current treatment position, the light pattern 24a, 24b indicates a next treatment position for the personal care device 2, and as the personal care device 2 moves towards the next treatment position, the projection of the light pattern 24a, 24b by the projection unit 22 is adjusted so that the light pattern 24a, 24b indicates substantially the same treatment position during the movement. This is illustrated with reference to Fig. 7. Fig. 7 is a simplified drawing that shows a projection unit 22 in a personal care device 2 that is in contact with the surface of the body part 70. The projection unit 22 is spaced from the surface of the body part 70 by a distance *dps* and is initially above a current treatment position 88.

The next treatment position 82 is shown a distance *d0* from the current treatment position 88. The projection unit 22 initially projects a light pattern 24 at an angle *α1* with respect to the vertical direction to the next treatment position 82, and angle *α1* is given by:

$$\alpha 1 = \arctan (d0/dps) \qquad (1)$$

As the personal care device 2 is moved along the surface towards the next treatment position 82, the processing unit 46 determines the distance moved from the measurements by the one or more movement sensors 44, and uses this to adjust the angle that the light pattern 24 is projected so that the light pattern 24 continues to indicate the next treatment position 82. Thus, at an arbitrary position 88x that is a distance x from the current treatment position 88 (where x is less than *d0*), the angle *α* at which the light pattern 24 is projected (labelled as light pattern 24x in Fig. 7) is given by:

$$\alpha(x) = \arctan ((d0 - x)/dps) \qquad (2)$$

Thus, the processing unit 46 determines x from the measurement signal(s), and determines *α* using equation (2). As the personal care device 2 moves towards the next treatment position 82, the angle *α* decreases in order to maintain the light pattern 24 at the next treatment position 82.

As noted above, the angle of projection of the light pattern 24 can be changed in a number of different ways. For example the position and/or angle of the projection unit 22 itself in the personal care device 2 can be adjusted using an actuation mechanism, or a position and/or angle of an optical component in the projection unit 22 can be adjusted.

In some embodiments, the processing unit 46 can control the projection unit 22 to change the projected light pattern 24 as the personal care device 2 moves towards the next treatment position 82. For example, the processing unit 46 can control the projection unit 22 to change the colour of the projected light pattern 24 as the personal care device 2 moves (e.g. initially the light pattern 24 can be green, and the pattern can become red as the personal care device 2 approaches the next treatment position 82. This embodiment can be used separately or in combination to the embodiment in which the angle of projection is adjusted due to the distance moved.

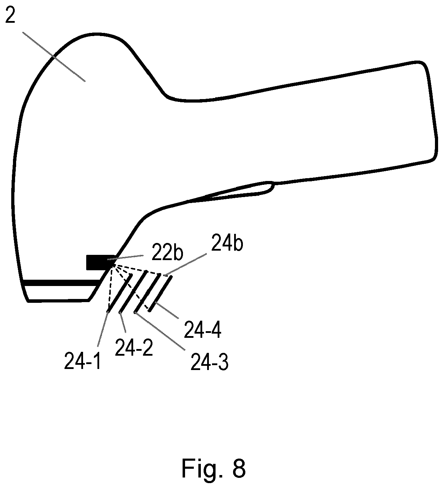

As an alternative to the dynamic projection angle-adjusting embodiment described above with reference to Fig. 7, certain embodiments can use a static arrangement of elements in the light pattern and selectively deactivate them as the personal care device 2 moves so that the visual guidance provided to the user is generally consistent throughout the movement of the personal care device 2 to the next treatment position. An example of this embodiment is shown in Fig. 8.

Fig. 8 shows a side view of a personal care device 2 similar to the embodiment shown in Fig. 1(b). In this embodiment, the projected light pattern 24b includes a number of parallel lines 24-1, 24-2, 24-3, 24-4 instead of a single line as in Fig. 5. Here, initially, the each of the lines 24-1 to 24-4 are projected, and line 24-4 (that is the furthest from the personal care device 2) indicates where the edge of the personal care device 2 should be in the next treatment position. As the personal care device 2 is moved, the processing unit 46 determines the amount of displacement, and the line projections are subsequently switched off from furthest 24-4 to nearest 24-1 to the personal care device 2, so that the furthest illuminated line 24 is approximately aligned with the edge of the personal care device 2 should be in the next treatment position. The deactivation of the last projected line 24-1 (i.e. the line nearest to the personal care device 2) can indicate to the user when to stop movement of the personal care device 2. The projection of a number of discrete lines can be achieved relatively easily using respective fixed light sources (such as LEDs) in the personal care device 2 that can be independently switched on and off. In a variation to the embodiment shown in Fig. 8, only one of the lines 24-1 to 24-4 may be projected at a given time, starting with the line 24-4 furthest from the personal care device 2, followed by the second furthest line 24-3, etc.

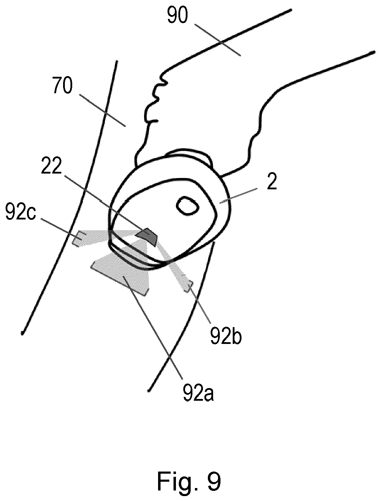

Fig. 9 illustrates another embodiment of a personal care device 2 according to the techniques described herein that is held by a hand 90 of a user. In this embodiment the personal care device 2 projects light patterns 92 in at least three directions, in front of the personal care device 2 (light pattern 92a), to the left of the personal care device 2 (light pattern 92b) and to the right of the personal care device 2 (light pattern 92c). These light patterns 92 may be projected by a single projection unit 22 or by respective projection units 22 in the personal care device 2. The light pattern 92a in front of the personal care device 2 can show the user where to move the personal care device 2 to for the next personal care operation, and the personal care device 2 can also simultaneously show light patterns 92b and 92c at the sides of the personal care device 2. This can provide users with an indication of how much to move the personal care device 2 to the left or right in case a new stroke is started. This can help to prevent untreated gaps between strokes.

As noted above, in some embodiments the projection unit 22 can be a projector or a pico-projector. A pico-projector can be used to project one or more lines on to the surface of the body part 70 according to the above embodiments, and/or it can project information on to the body part 70, such as an arrow indicating a direction that the personal care device 2 should be moved to the next treatment position, numbers relating to the personal care operation (e.g. the number of areas of the body treated so far, a number remaining, etc.). In some embodiments, the information projected using the pico-projector can be 'gamified' to help encourage the user use the personal care device 2 appropriately to perform the personal care operation. In another embodiment, the pico-projector can project a grid-like structure on to the surface of the body part. In some embodiments the elements in the grid-like structure are generally the same size as treatment areas. During a personal care session using a personal care device with a remote camera-based simultaneous localisation and mapping (SLAM) solution, a skin area map can be generated while in motion, with the skin area map comprising information about treatment coverage (including treated areas, over-treated areas, missed areas, under-treated areas, etc.). This information can be projected into the grid-like structure to show the user how effective the personal care operation was. In some embodiments different colours can be projected into the grid elements to illustrate the relevant information for the grid element (e.g. green for a successfully treated area, red for an over-treated area, no colour for a missed area, and yellow for an under-treated area).

In further embodiments, based on analysis of the movement sensor measurement signals, the processing unit 46 may determine feedback for the user based on whether the user moves the personal care device too far when moving to the next treatment position (which means that there is a gap between successive treatment areas), or not far enough (which means that there is too much overlap between successive treatment areas).

In related embodiments, based on analysis of the movement sensor measurement signals, the processing unit 46 may determine adjustments to the position of the next projected light pattern to improve the likelihood that the user moves the personal care device by the correct amount. In particular, the processing unit 46 can implement a learning control module or algorithm to adjust or adapt the position of the next projected light pattern. Thus the processing unit 46 can process the movement sensor signal(s) to determine an amount of movement of the personal care device from a current treatment position at which the personal care operation is performed by the personal care device to a next treatment position at which a next personal care operation is performed by the personal care device. This can be repeated a number of times for a number of movements of the personal care device until the processing unit 46 has sufficient data to determine an adjustment to the positioning of the light pattern indicating the next treatment position. The processing unit 46 then compares each determined amount of movement of the personal care device from a current treatment position to the next treatment position to an amount of movement expected between these treatment positions. Based on the result of this comparison, the processing unit 46 can control the projection unit 22 to adjust the projection position of a light pattern indicative of a next treatment position on the body. For example the comparison may indicate that the user consistently moves the personal care device too far by an amount D when the light pattern is projected to the edge of the next treatment area, and so the processing unit 46 can control the projection unit 22 to project the light pattern closer to the personal care device (in some implementations closer by a distance D, or alternatively closer by a distance that is less than D so that the processing unit 46 can iterate the adjustment over time). If the user consistently moves the personal care device too far, moving the projected light pattern closer to the personal care device may lead the user to moving the personal care device by a shorter (and more appropriate) distance each time. On the other hand, if the comparison indicates that the distance the user moves the personal care device is consistently short of the required distance by an amount E when the light pattern is projected to the edge of the next treatment area, the processing unit 46 can control the projection unit 22 to project the light pattern further away from the personal care device (in some implementations further by a distance E, or alternatively further away by a distance that is less than E so that the processing unit 46 can iterate the adjustment over time). If the user consistently doesn't move the personal care device far enough, moving the projected light pattern further away from the personal care device may lead the user to moving the personal care device by a longer (and more appropriate) distance each time. The learning process outlined above can be repeated until an optimal adjustment to the position of the projected light pattern is found. It will be appreciated that the optimal adjustment may be different for different body parts, and/or for different strokes on body parts (e.g. a user may find it easier to comply with the visual guidance when performing a personal care operation sequentially moving the personal care device down the leg, but consistently moves the personal care device too far between sequential treatments when moving the personal care device back up the leg).

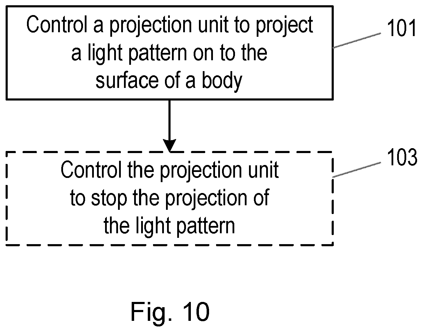

Fig. 10 is a flow chart illustrating a method of operating an apparatus to provide visual guidance to a user of a personal care device 2 according to various embodiments. In a first step, step 101, when the personal care device 2 is at a current treatment position on a body, the processing unit 46 controls a projection unit 22 to project a light pattern on to the surface of a body. The light pattern is projected to a projection position that is indicative of a next treatment position on the body to which the personal care device is to be moved by the user from the current treatment position. This light pattern provides visual guidance to the user of the personal care device 2 to show the user how far to move the personal care device 2 to the next treatment position.

In step 103, the processing unit 46 controls the projection unit 22 to stop the projection of the light pattern on to the body. As noted above, step 103 may occur when the processing unit 46 determines that the personal care device is being moved from the current treatment position, determines that the user has released a user control 20 used to trigger the personal care operation, determines that the user has released a control used by the user to activate the visual guidance operation, determines that the user has actuated a control to deactivate the visual guidance operation, or determines that the visual guidance has been projected for a predetermined amount of time.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. An apparatus (42) configured for use with a personal care device (2), wherein the apparatus (42) is configured to provide visual guidance to a user on movement of the personal care device (2) across a body of a subject, wherein the personal care device (2) is configured to perform a personal care operation on a plurality of successive treatment areas of the body by successively moving the personal care device from a respective one of a plurality of current treatment positions on the body corresponding with a respective one of said successive treatment areas to a next treatment position on the body corresponding with a next one of said successive treatment areas and performing the personal care operation to said next one of said successive treatment areas while maintaining the personal care device in said next one of said treatment positions, the apparatus (42) comprising:

   a projection unit (22) configured to project a light pattern on to a surface of the body; and
   a processing unit (46) configured to:
   control the projection unit (22) to project the light pattern on to the surface of the body in a projection position;

   **characterized in that** the projection position is indicative of the next treatment position on the body to which the personal care device (2) is to be moved by the user from the current treatment position on the body.

2. An apparatus (42) as claimed in claim 1, wherein the projection position is such that, seen in a direction in which the personal care device (2) is to be moved by the user, the treatment area corresponding with the next treatment position seamlessly connects to the treatment area corresponding with the current treatment position.

3. An apparatus (42) as claimed in claim 1 or 2, wherein the processing unit (46) is configured to control the projection unit to project the light pattern in the projection position after the personal care operation has been performed at the current treatment position of the personal care device (2) on the body.

4. An apparatus (42) as claimed in claim 1 or 2, wherein the processing unit (46) is configured to control the projection unit (22) to project the light pattern in response to a first input from the user.

5. An apparatus (42) as claimed in any of claims 1-4, wherein the processing unit (46) is configured to control the projection unit (22) to stop the projection of the light pattern in response to a second input from the user.

6. An apparatus (42) as claimed in any of claims 1-4, wherein the processing unit (46) is further configured to:

   receive a signal from a movement sensor (44) associated with the personal care device (2);
   process the received signal to determine when the personal care device (2) starts to move from the current treatment position on the body; and
   adjust the operation of the projection unit (22) when the personal care device (2) is determined to be starting to move from the current treatment position.

7. An apparatus (42) as claimed in claim 6, wherein the processing unit (46) is configured to adjust the operation of the projection unit (22) by controlling the projection unit (22) to deactivate the projection of the light pattern when the personal care device (2) starts to move from the current treatment position.

8. An apparatus (42) as claimed in claim 6, wherein the processing unit (46) is configured to control the projection unit (22) based on the signal received from the movement sensor such that the projection position of the projected light pattern is substantially static relative to the surface of the body in response to the movement of the personal care device (2) from the current treatment position to the next treatment position.

9. An apparatus (42) as claimed in any of claims 1-8, wherein the light pattern comprises one or more lines indicative of a position of an edge of the personal care device (2) on the surface of the body associated with the next treatment position of the personal care device (2) on the body.

10. An apparatus (42) as claimed in any of claims 1-8, wherein the light pattern is configured to highlight the treatment area of the body corresponding with the next treatment position of the personal care device (2) on the body.

11. An apparatus (42) as claimed in any of claims 1-10, wherein the processing unit is further configured to:

i. receive a signal from a movement sensor (44) associated with the personal care device (2);
ii. process the received signal to determine an amount of movement of the personal care device (2) from a first treatment position at which a first personal care operation is performed by the personal care device (2) to a next, second, treatment position at which a second personal care operation is performed by the personal care device (2);
iii. repeat ii for a plurality of movements of the personal care device (2) from first treatment positions to next, second, treatment positions; and
iv. compare each determined amount of movement to an expected amount of movement from each first treatment position to the respective next second treatment position;

wherein the processing unit (46) is configured to control the projection unit (22) to project the light pattern on to the surface of the body in a projection position indicative of a next treatment position on the body to which the personal care device (2) is to be moved based on the result of the comparison.

12. An apparatus (42) as claimed in any of claims 1-11, wherein:

i. the apparatus (42) further comprises at least one further projection unit (22) configured to project a further light pattern on to a different part of the body; or
ii. the projection unit (22) is further configured to project a further light pattern on to a different part of the body.

13. A personal care device (2) configured to perform a personal care operation on a body of a subject, the personal care device (2) comprising:
an apparatus (42) as claimed in any of claims 1-12 configured to provide visual guidance to a user of the personal care device (2) on movement of the personal care device (2) across the body of the subject.

14. A method of operating an apparatus to provide visual guidance to a user of a personal care device, wherein the visual guidance is for guiding movement of the personal care device across a body of a subject by the user, wherein the personal care device is configured to perform a personal care operation on a plurality of successive treatment areas of the body by successively moving the personal care device from a respective one of a plurality of successive current treatment positions on the body corresponding with a respective one of said successive treatment areas to a next treatment position on the body corresponding with a next one of said successive treatment areas and performing the personal care operation to said next one of said successive treatment areas while maintaining the personal care device in said next one of said successive treatment positions, the method comprising:
controlling (101) a projection unit in the apparatus to project a light pattern on to the surface of the body in a projection position;
**characterized in that** the projection position is indicative of the next treatment position on the body to which the personal care device is to be moved by the user from the current treatment position on the body.

15. A computer program product comprising a computer readable medium having computer readable code embodied therein, the computer readable code being configured such that, on execution by a suitable computer or processor, the computer or processor is caused to perform the method of claim 14.

**Patentansprüche**

1. Einrichtung (42), die zur Verwendung mit einer Körperpflegevorrichtung (2) konfiguriert ist, wobei die Einrichtung (42) konfiguriert ist, um einem Benutzer eine visuelle Führung beim Bewegen der Körperpflegevorrichtung (2) über den Körper eines Subjekts bereitzustellen, wobei die Körperpflegevorrichtung (2) konfiguriert ist, um einen Körperpflegevorgang an einer Vielzahl von aufeinanderfolgenden Behandlungsbereichen des Körpers durch aufeinanderfolgendes Bewegen der Körperpflegevorrichtung von einer jeweiligen einer Vielzahl von aktuellen Behandlungspositionen am Körper, die einem jeweiligen der aufeinanderfolgenden Behandlungsbereiche entspricht, zu einer nächsten Behandlungsposition am Körper, die einem nächsten der aufeinanderfolgenden Behandlungsbereiche entspricht, und Durchführen des Körperpflegevorgangs an dem nächsten der aufeinanderfolgenden Behandlungsbereiche durchzuführen, während die Körperpflegevorrichtung in der nächsten der Behandlungspositionen gehalten wird, wobei die Einrichtung (42) umfasst:

eine Projektionseinheit (22), die konfiguriert ist, um ein Lichtmuster auf eine Oberfläche des Körpers zu projizieren; und
eine Verarbeitungseinheit (46), die konfiguriert ist, um:

die Projektionseinheit (22) zu steuern, um das Lichtmuster in einer Projektionsposition auf die Oberfläche des Körpers zu projizieren;
**dadurch gekennzeichnet, dass** die Projektionsposition die nächste Behandlungsposition am Körper angibt, zu der die Körperpflegevorrichtung (2) von dem Benutzer aus der aktuellen Behandlungsposition am Körper zu bewegen ist.

2. Einrichtung (42) nach Anspruch 1, wobei die Projektionsposition so ist, dass, in einer Richtung gesehen, in der die Körperpflegevorrichtung (2) vom Benutzer zu bewegen ist, der Behandlungsbereich, welcher der nächsten Behandlungsposition entspricht, nahtlos an den Behandlungsbereich anschließt, welcher der aktuellen Behandlungsposition entspricht.

3. Einrichtung (42) nach Anspruch 1 oder 2, wobei die Verarbeitungseinheit (46) konfiguriert ist, um die Projektionseinheit zu steuern, um das Lichtmuster in der Projektionsposition zu projizieren, nachdem der Körperpflegevorgang an der aktuellen Behandlungsposition der Körperpflegevorrichtung (2) am Körper durchgeführt worden ist.

4. Einrichtung (42) nach Anspruch 1 oder 2, wobei die Verarbeitungseinheit (46) konfiguriert ist, um die Projektionseinheit (22) zu steuern, um das Lichtmuster als Reaktion auf eine erste Eingabe des Benutzers zu projizieren.

5. Einrichtung (42) nach einem der Ansprüche 1-4, wobei die Verarbeitungseinheit (46) konfiguriert ist, um die Projektionseinheit (22) zu steuern, um die Projektion des Lichtmusters als Reaktion auf eine zweite Eingabe des Benutzers zu stoppen.

6. Einrichtung (42) nach einem der Ansprüche 1-4, wobei die Verarbeitungseinheit (46) weiter konfiguriert ist, um:

ein Signal von einem Bewegungssensor (44) zu empfangen, der mit der Körperpflegevorrichtung (2) verknüpft ist;
das empfangene Signal zu verarbeiten, um zu bestimmen, wann die Körperpflegevorrichtung (2) beginnt, sich von der aktuellen Behandlungsposition am Körper wegzubewegen; und
den Betrieb der Projektionseinheit (22) anzupassen, wenn bestimmt wird, dass die Körperpflegevorrichtung (2) beginnt, sich von der aktuellen Behandlungsposition wegzubewegen.

7. Einrichtung (42) nach Anspruch 6, wobei die Verarbeitungseinheit (46) konfiguriert ist, um den Betrieb der Projektionseinheit (22) durch Steuern der Projektionseinheit (22) anzupassen, um die Projektion des Lichtmusters zu deaktivieren, wenn die Körperpflegevorrichtung (2) beginnt, sich von der aktuellen Behandlungsposition wegzubewegen.

8. Einrichtung (42) nach Anspruch 6, wobei die Verarbeitungseinheit (46) konfiguriert ist, um die Projektionseinheit (22) basierend auf dem vom Bewegungssensor empfangenen Signal zu steuern, sodass die Projektionsposition des projizierten Lichtmusters als Reaktion auf die Bewegung der Körperpflegevorrichtung (2) von der aktuellen Behandlungsposition zur nächsten Behandlungsposition in Bezug auf die Körperoberfläche im Wesentlichen statisch ist.

9. Einrichtung (42) nach einem der Ansprüche 1-8, wobei das Lichtmuster eine oder mehrere Linien umfasst, die eine Position einer Kante der Körperpflegevorrichtung (2) auf der Körperoberfläche angeben, die mit der nächsten Behandlungsposition der Körperpflegevorrichtung (2) auf dem Körper ist.

10. Einrichtung (42) nach einem der Ansprüche 1-8, wobei das Lichtmuster konfiguriert ist, um den Behandlungsbereich des Körpers, welcher der nächsten Behandlungsposition der Körperpflegevorrichtung (2) am Körper entspricht, hervorzuheben.

**11.** Einrichtung (42) nach einem der Ansprüche 1-10, wobei die Verarbeitungseinheit weiter konfiguriert ist, um:

i. ein Signal von einem Bewegungssensor (44) zu empfangen, der mit der Körperpflegevorrichtung (2) verknüpft ist;

ii. das empfangenen Signal zu verarbeiten, um einen Umfang einer Bewegung der Körperpflegevorrichtung (2) von einer ersten Behandlungsposition, an der ein erster Körperpflegevorgang durch die Körperpflegevorrichtung (2) durchgeführt wird, zu einer nächsten, zweiten Behandlungsposition, an der ein zweiter Körperpflegevorgang durch die Körperpflegevorrichtung (2) durchgeführt wird, zu bestimmen;

iii. ii für eine Vielzahl von Bewegungen der Körperpflegevorrichtung (2) von ersten Behandlungspositionen zu nächsten, zweiten Behandlungspositionen zu wiederholen; und

iv. jeden bestimmten Umfang einer Bewegung mit einem erwarteten Umfang einer Bewegung von jeder ersten Behandlungsposition zur jeweils nächsten zweiten Behandlungsposition zu vergleichen;

wobei die Verarbeitungseinheit (46) konfiguriert ist, um die Projektionseinheit (22) zu steuern, um das Lichtmuster auf die Oberfläche des Körpers in einer Projektionsposition zu projizieren, die eine nächste Behandlungsposition auf dem Körper angibt, in welche die Körperpflegevorrichtung (2) basierend auf dem Ergebnis des Vergleichs zu bewegen ist.

**12.** Einrichtung (42) nach einem der Ansprüche 1-11, wobei:

i. die Einrichtung (42) weiter mindestens eine weitere Projektionseinheit (22) umfasst, die konfiguriert ist, um ein weiteres Lichtmuster auf einen anderen Teil des Körpers zu projizieren; oder

ii. die Projektionseinheit (22) weiter konfiguriert ist, um ein weiteres Lichtmuster auf einen anderen Teil des Körpers zu projizieren.

**13.** Körperpflegevorrichtung (2), die konfiguriert ist, um einen Körperpflegevorgang durchzuführen an einem Körper eines Subjekts, wobei die Körperpflegevorrichtung (2) umfasst:
eine Einrichtung (42) nach einem der Ansprüche 1-12, die konfiguriert ist, um einem Benutzer der Körperpflegevorrichtung (2) beim Bewegen der Körperpflegevorrichtung (2) über den Körper des Subjekts eine visuelle Führung bereitzustellen.

**14.** Verfahren zum Betreiben einer Einrichtung, um einem Benutzer einer Körperpflegevorrichtung visuelle Führung bereitzustellen, wobei die visuelle Führung zum Führen einer Bewegung der Körperpflegevorrichtung über einen Körper eines Subjekts durch den Benutzer dient, wobei die Körperpflegevorrichtung konfiguriert ist, um einen Körperpflegevorgang an einer Vielzahl von aufeinanderfolgenden Behandlungsbereichen des Körpers durch aufeinanderfolgendes Bewegen der Körperpflegevorrichtung von einer jeweiligen einer Vielzahl von aufeinanderfolgenden aktuellen Behandlungspositionen am Körper, die einem jeweiligen der aufeinanderfolgenden Behandlungsbereiche entspricht, zu einer nächsten Behandlungsposition am Körper, die einem nächsten der aufeinanderfolgenden Behandlungsbereiche entspricht, und Durchführen des Körperpflegevorgangs an dem nächsten der aufeinanderfolgenden Behandlungsbereiche durchzuführen, während die Körperpflegevorrichtung in der nächsten der aufeinanderfolgenden Behandlungspositionen gehalten wird, wobei das Verfahren umfasst:

Steuern (101) einer Projektionseinheit in der Einrichtung, um ein Lichtmuster in einer Projektionsposition auf die Oberfläche des Körpers zu projizieren;
**dadurch gekennzeichnet, dass** die Projektionsposition die nächste Behandlungsposition am Körper angibt, zu der die Körperpflegevorrichtung von dem Benutzer aus der aktuellen Behandlungsposition am Körper zu bewegen ist.

**15.** Computerprogrammprodukt, das ein computerlesbares Medium umfasst, das einen darin eingebetteten computerlesbaren Code aufweist, wobei der computerlesbare Code konfiguriert ist, sodass bei Ausführung durch einen geeigneten Computer oder Prozessor der Computer oder Prozessor veranlasst wird, das Verfahren nach Anspruch 14 durchzuführen.

**Revendications**

**1.** Appareil (42) configuré pour être utilisé avec un dispositif de soins personnels (2), dans lequel l'appareil (42) est configuré pour fournir un guidage visuel à un utilisateur lors d'un mouvement du dispositif de soins personnels (2) sur le corps d'un sujet, dans lequel le dispositif de soins personnels (2) est configuré pour effectuer une opération de soins personnels sur une pluralité de zones de traitement successives du corps en déplaçant successivement le dispositif de soins personnels d'une position de traitement respective d'une pluralité de positions de traitement actuelles sur le corps correspondant à une zone de traitement respective desdites zones de traitement successives jusqu'à une prochaine position de traitement sur le corps correspondant à une prochaine zone de traitement desdites zones de traitement successives et en effectuant l'opération de soins personnels sur ladite prochaine zone de traitement desdites zones de traitement successives tout en maintenant le dispositif de soins personnels dans ladite prochaine position de traitement desdites positions de traitement, l'appareil (42) comprenant :

une unité de projection (22) configurée pour projeter un motif lumineux sur une surface du corps ; et
une unité de traitement (46) configurée pour :

commander l'unité de projection (22) pour projeter le motif lumineux sur la surface du corps dans une position de projection ;
**caractérisé en ce que** la position de projection indique la prochaine position de traitement sur le corps jusqu'à laquelle le dispositif de soins personnels (2) doit être déplacé par l'utilisateur à partir de la position de traitement actuelle sur le corps.

2. Appareil (42) selon la revendication 1, dans lequel la position de projection est telle que, vue dans une direction dans laquelle le dispositif de soins personnels (2) doit être déplacé par l'utilisateur, la zone de traitement correspondant à la prochaine position de traitement se connecte de manière transparente à la zone de traitement correspondant à la position de traitement actuelle.

3. Appareil (42) selon la revendication 1 ou 2, dans lequel l'unité de traitement (46) est configurée pour commander l'unité de projection pour projeter le motif lumineux dans la position de projection après que l'opération de soins personnels a été effectuée à la position de traitement actuelle du dispositif de soins personnels (2) sur le corps.

4. Appareil (42) selon la revendication 1 ou 2, dans lequel l'unité de traitement (46) est configurée pour commander l'unité de projection (22) pour projeter le motif lumineux à la suite d'une première entrée de l'utilisateur.

5. Appareil (42) selon l'une quelconque des revendications 1-4, dans lequel l'unité de traitement (46) est configurée pour commander l'unité de projection (22) pour arrêter la projection du motif lumineux à la suite d'une seconde entrée de l'utilisateur.

6. Appareil (42) selon l'une quelconque des revendications 1-4, dans lequel l'unité de traitement (46) est en outre configurée pour :

recevoir un signal en provenance d'un capteur de mouvement (44) associé au dispositif de soins personnels (2) ;
traiter le signal reçu pour déterminer quand le dispositif de soins personnels (2) commence à se déplacer à partir de la position de traitement actuelle sur le corps ; et
ajuster le fonctionnement de l'unité de projection (22) lorsqu'il est déterminé que le dispositif de soins personnels (2) commence à se déplacer à partir de la position de traitement actuelle.

7. Appareil (42) selon la revendication 6, dans lequel l'unité de traitement (46) est configurée pour ajuster le fonctionnement de l'unité de projection (22) en commandant l'unité de projection (22) pour désactiver la projection du motif lumineux lorsque le dispositif de soins personnels (2) commence à se déplacer à partir de la position de traitement actuelle.

8. Appareil (42) selon la revendication 6, dans lequel l'unité de traitement (46) est configurée pour commander l'unité de projection (22) sur la base du signal reçu du capteur de mouvement de telle sorte que la position de projection du motif lumineux projeté soit sensiblement statique par rapport à la surface du corps à la suite du mouvement du dispositif de soins personnels (2) de la position de traitement actuelle à la position de traitement suivante.

9. Appareil (42) selon l'une quelconque des revendications 1-8, dans lequel le motif lumineux comprend une ou plusieurs lignes indiquant une position d'un bord du dispositif de soins personnels (2) sur la surface du corps associée à la prochaine position de traitement du dispositif de soins personnels (2) sur le corps.

**10.** Appareil (42) selon l'une quelconque des revendications 1-8, dans lequel le motif lumineux est configuré pour mettre en évidence la zone de traitement du corps correspondant à la prochaine position de traitement du dispositif de soins personnels (2) sur le corps.

**11.** Appareil (42) selon l'une quelconque des revendications 1-10, dans lequel l'unité de traitement est en outre configurée pour :

   i. recevoir un signal en provenance d'un capteur de mouvement (44) associé au dispositif de soins personnels (2) ;
   ii. traiter le signal reçu pour déterminer une quantité de mouvement du dispositif de soins personnels (2) à partir d'une première position de traitement à laquelle une première opération de soins personnels est effectuée par le dispositif de soins personnels (2) jusqu'à une seconde prochaine position de traitement à laquelle une seconde opération de soins personnels est effectuée par le dispositif de soins personnels (2) ;
   iii. répéter ii pour une pluralité de mouvements du dispositif de soins personnels (2) des premières positions de traitement aux secondes prochaines positions de traitement ; et
   iv. comparer chaque quantité déterminée de mouvement à une quantité attendue de mouvement de chaque première position de traitement à la seconde prochaine position de traitement respective ;

dans lequel l'unité de traitement (46) est configurée pour commander l'unité de projection (22) pour projeter le motif lumineux sur la surface du corps dans une position de projection indiquant une prochaine position de traitement sur le corps jusqu'à laquelle le dispositif de soins personnels (2) doit être déplacé sur la base du résultat de la comparaison.

**12.** Appareil (42) selon l'une quelconque des revendications 1-11, dans lequel :

   i. l'appareil (42) comprend en outre au moins une unité de projection supplémentaire (22) configurée pour projeter un motif lumineux supplémentaire sur une partie différente du corps ; ou
   ii. l'unité de projection (22) est en outre configurée pour projeter un motif lumineux supplémentaire sur une partie différente du corps.

**13.** Dispositif de soins personnels (2) configuré pour effectuer une opération de soins personnels sur un corps d'un sujet, le dispositif de soins personnels (2) comprenant :
un appareil (42) tel que revendiqué dans l'une quelconque des revendications 1-12 configuré pour fournir un guidage visuel à un utilisateur du dispositif de soins personnels (2) lors du mouvement du dispositif de soins personnels (2) d'un côté à l'autre du corps du sujet.

**14.** Procédé de fonctionnement d'un appareil pour fournir un guidage visuel à un utilisateur d'un dispositif de soins personnels, dans lequel le guidage visuel est destiné à guider un mouvement du dispositif de soins personnels d'un côté à l'autre du corps d'un sujet par l'utilisateur, dans lequel le dispositif de soins personnels est configuré pour effectuer une opération de soins personnels sur une pluralité de zones de traitement successives du corps en déplaçant successivement le dispositif de soins personnels d'une position de traitement respective d'une pluralité de positions de traitement actuelles successives sur le corps correspondant à une zone de traitement respective desdites zones de traitement successives jusqu'à une prochaine position de traitement sur le corps correspondant à une prochaine zone de traitement desdites zones de traitement successives et en effectuant l'opération de soins personnels sur ladite prochaine zone de traitement desdites zones de traitement successives tout en maintenant le dispositif de soins personnels dans ladite prochaine position de traitement desdites positions de traitement successives, le procédé comprenant :

   la commande (101) d'une unité de projection dans l'appareil pour projeter un motif lumineux sur la surface du corps dans une position de projection ;
   **caractérisé en ce que** la position de projection indique la prochaine position de traitement sur le corps jusqu'à laquelle le dispositif de soins personnels doit être déplacé par l'utilisateur à partir de la position de traitement actuelle sur le corps.

**15.** Produit de programme informatique comprenant un support lisible par ordinateur présentant un code lisible par ordinateur incorporé dans celui-ci, le code lisible par ordinateur étant configuré de telle sorte que, lors de son exécution par un ordinateur, ou un processeur, approprié, l'ordinateur, ou le processeur, soit amené à effectuer le procédé selon la revendication 14.

Fig. 1a

EP 4 157 125 B1

Fig. 1b

Fig. 2

Fig. 3

Fig. 4

(b)

(a)

Fig. 5

Fig. 6

Fig. 7

EP 4 157 125 B1

Fig. 8

Fig. 9

Control a projection unit to project
a light pattern on to the
surface of a body

101

Control the projection unit
to stop the projection of
the light pattern

103

Fig. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019224273 A1